**Europäisches Patentamt**

**European Patent Office**

**Office européen des brevets**

(19)

(11) Numéro de publication : **0 409 703 B1**

(12) # FASCICULE DE BREVET EUROPEEN

(45) Date de publication du fascicule du brevet :
**22.09.93 Bulletin 93/38**

(51) Int. Cl.⁵ : **A61L 2/18**, G02C 13/00

(21) Numéro de dépôt : **90402028.6**

(22) Date de dépôt : **13.07.90**

(54) Préparation pour la décontamination et le nettoyage des lentilles de contact, et son utilisation.

(30) Priorité : **18.07.89 FR 8909620**

(43) Date de publication de la demande :
**23.01.91 Bulletin 91/04**

(45) Mention de la délivrance du brevet :
**22.09.93 Bulletin 93/38**

(84) Etats contractants désignés :
**AT BE CH DE DK ES GB IT LI LU NL SE**

(56) Documents cités :
**US-A- 3 954 965**

(73) Titulaire : **BOURGEOIS S.A.**
**Morbier**
**F-39400 Morez (FR)**

(72) Inventeur : **Legoffic, François**
**42, rue Jean Georget**
**F-92140 Clamart (FR)**
Inventeur : **Klagba, Wisdom**
**1 place des Onze Arpents**
**F-94800 Villejuif (FR)**

(74) Mandataire : **Cournarie, Michèle et al**
**Office Blétry 2, boulevard de Strasbourg**
**F-75010 Paris (FR)**

EP 0 409 703 B1

## Description

L'invention concerne une préparation pour la décontamination et le nettoyage des lentilles de contact, et son utilisation.

Parmi les nombreuses solutions qui ont été proposées pour la décontamination des lentilles de contact, deux sont particulièrement utilisées :

- le trempage dans de l'eau oxygénée suivi de la dégradation de l'agent décontaminant en excès ou inclus dans la lentille. Cette dégradation se fait en présence d'un catalyseur, généralement du platine,
- le trempage dans une solution d'hypochlorite, celle-ci étant formée par décomposition dans l'eau d'un composé stable, le plus souvent une chloramine. Un rinçage est ensuite effectué avec une solution isotonique de chlorure de sodium dans l'eau (sérum physiologique).

Une telle opération en deux étapes est longue et fastidieuse pour l'utilisateur et, de plus, dans le cas de l'utilisation d'hypochlorite, l'odeur caractéristique de ce dernier peut rebuter l'utilisateur.

On a donc besoin d'un procédé simple et efficace de décontamination des lentilles de contact, utilisant des agents de décontamination inodores et à effet rapide, et que l'on puisse avantageusement associer à des agents de nettoyage classiques (détergents).

De telles solutions ont été proposées, comme par exemple une composition contenant du triméthoprime, de l'alcool benzylique, de l'acide éthylènediamine tétraacétique et de la polyvinylpyrrolidone (voir par exemple les brevets US 4 560 491, EP 175 490 ou US 4 551 461 au nom de Sherman Laboratories INC.). Celles-ci présentent, bien entendu, l'énorme inconvénient de contenir un antibiotique tel que le triméthoprime qui, s'il reste inclus dans la lentille à une faible concentration, inférieure à celle nécessaire pour tuer les microorganismes, va sélectionner des bactéries qui deviendront de plus en plus résistantes à cet antibiotique.

Une composition de nettoyage et de désinfection contenant de l'anhydride sulfureux appelé aussi dioxyde de soufre, de l'acide benzoïque, de l'acide citrique et du carbonate de sodium (voir par exemple le brevet EP. 242 998) possède sans nul doute une odeur désagréable et une certane agressivité.

Une solution extrêmement efficace pour résoudre les problèmes de désinfection, de déprotéinisation, de désionisation et de nettoyage des lentilles de contact est proposée par la présente invention. Elle consiste à immerger les lentilles de contact dans un solvant aqueux dans lequel est dissous, en tant qu'ingrédient actif de décontamination, de l'alcool dichloro-2,4 benzylique, agent antiseptique connu et déjà utilisé pour la désinfection de la gorge, par exemple.

Le solvant aqueux utilisé peut être de l'eau préférablement purifiée, amenée à un pH compatible avec la physiologie de l'oeil, ou un mélange d'une telle eau et d'un solvant miscible à l'eau, ou, de préférence, une solution isotonique de chlorure de sodium dans l'eau.

L'ingrédient actif est avantageusement dissous dans le solvant aqueux au moment de l'emploi.

La présente invention fournit également une préparation solide pour la décontamination de lentilles de contact, contenant, en tant qu'ingrédient actif de décontamination, de l'alcool dichloro-2,4 benzylique, dans la proportion préférée de 2 à 5% en poids.

Cette préparation contient en outre avantageusement de l'acide benzoïque, ou du benzoate de sodium, ou un autre sel de métal alcalin ou alcalino-terreux de l'acide benzoïque, essentiellement en tant qu'agent lubrifiant favorisant une bonne mise en forme galénique de la préparation solide, en particulier lorsque celle-ci est présentée en comprimés; toutefois, cet adjuvant a aussi complémentairement un effet antiseptique, notamment antimycosique.

Cette préparation solide peut également contenir un agent détergent et un agent séquestrant afin de lui donner des propriétés de nettoyage. Les détergents et séquestrants usuels, ne présentant pas de contre-indications au niveau oculaire, peuvent être utilisés et on citera, à titre d'exemple, le laurylsulfate de sodium et l'acide éthylènediaminetétraacétique (EDTA).

L'invention fournit également une solution de décontamination de lentilles de contact, obtenue par dissolution dans un solvant aqueux, et de préférence dans une solution isotonique de chlorure de sodium dans l'eau, de la préparation solide selon l'invention.

Pour que la solution ainsi préparée soit efficace et permette de décontaminer des lentilles de contact en un temps relativement court (10 minutes ou moins), la concentration en alcool dichloro-2,4 benzylique doit être de 0,2 à 0,5 mg par ml de solution finale. Une concentration inférieure à 0,2 mg/ml n'est pas suffisante pour une décontamination fiable alors qu'une concentration supérieure à 0,5 mg n'apporte pas d'avantages particuliers.

La préparation solide est donc formulée de façon à obtenir la concentration voulue dans la solution finale. Le volume de la solution de décontamination est généralement de quelques ml, 5 à 10 par exemple, et la dose de préparation solide que l'on y dissout contient la quantité appropriée d'alcool dichloro-2,4 benzylique. La concentration de l'ingrédient actif dans la dose dépend bien évidemment de la quantité des autres ingrédients.

La proportion de l'agent lubrifiant par rapport à l'alcool dichloro-2,4 benzylique peut varier sans grande modification du pouvoir antiseptique de la préparation; le rapport en poids de ces deux constituants peut être de 1/1 à 6/1.

En plus de l'agent de lubrification (benzoate de sodium par exemple), du détergent (laurylsulfate de sodium par exemple) et de l'agent séquestrant (EDTA par exemple), la préparation solide contient généralement un agent mouillant pour améliorer le contact entre la solution de décontamination et les lentilles ainsi que des agents favorisant le délitement de la préparation solide dans la solution isotonique, en particulier lorsque la préparation solide est présentée sous forme de comprimés. A titre d'exemple de l'agent mouillant, on peut citer la polyvinylpyrrolidone et, en tant qu'agents de délitement, on peut utiliser de l'acide citrique associé à de l'hydrogénocarbonate de sodium.

La préparation solide peut être présentée soit sous forme de poudre libre, soit sous forme de granulés, soit sous forme de comprimés. Eu égard au caractère hygroscopique d'une telle préparation, la poudre libre et les granulés sont présentés en doses individuelles dans des sachets étanches et les comprimés sont conservés par exemple en blisters ou, mieux, dans des emballages en feuilles d'aluminium.

Les exemples qui suivent illustrent la préparation et l'utilisation d'une préparation solide selon l'invention, étant entendu que la composition précise donnée ne l'est qu'à titre indicatif et peut être modifiée.

EXEMPLE 1.

EXEMPLE 1.

Préparation sous forme de poudre libre.

| | | |
|---|---|---|
| Alcool dichloro-2,4 benzylique | 3 | g |
| Benzoate de sodium | 9,2 | g |
| EDTA | 7,5 | g |
| Laurylsulfate de sodium | 3,2 | g |
| Acide citrique | 35 | g |
| Hydrogénocarbonate de sodium | 41 | g |
| Polyvinylpyrrolidone | 1,10 | g |
| | 100 | g |

La poudre ainsi obtenue est répartie en sachets étanches contenant chacun 100 mg de poudre, soit 3 mg d'alcool dichloro-2,4 benzylique par sachet destiné à être dissous dans 5 à 10ml de sérum physiologique.

EXEMPLE 2

Préparation de comprimés.

On prépare un mélange effervescent de 70 g d'acide citrique et 82 g d'hydrogénocarbonate de sodium puis on prépare un liquide de mouillage en dissolvant 2,20 g de polyvinylpyrrolidone puis 6,0 g d'alcool dichloro-2,4 benzylique dans 25 ml d'alcool isopropylique. On mouille le mélange effervescent avec ce liquide de mouillage puis on sèche 12 heures à l'étuve à 45°C pour chasser l'alcool isopropylique. On granule le mélange dans un granulateur oscillant muni d'une grille ayant une ouverture de maille de 600 $\mu$m. Puis les granulés sont introduits dans un mélangeur à tambour avec les ingrédients restants (18,4 g de benzoate de sodium, 15,0g de EDTA et 6,4 g de laurylsulfate de sodium). Le mélange est homogénéisé pendant 20 mn et a la même composition que la poudre de l'exemple 1. On comprime ensuite le mélange pour former des comprimés de 100 mg chacun, ayant une dureté radiale (pression maximale supportée, exercée à la périphérie du comprimé) voisine de 2 kgp/cm$^2$ (environ 2.10$^5$ Pa), qui augmente sensiblement après quelques jours.

Un comprimé ainsi préparé se dissout en environ 3 mn dans 7 ml de sérum physiologique, en donnant une concentration de 14,3 mg de préparation par ml de solution (soit 0,43 mg d'alcool dichloro-2,4 benzylique par ml de solution).

EXEMPLE 3.

Pouvoir antiseptique (décontaminant) de la préparation selon l'invention.

L'étude est faite in vitro selon les règles de la Pharmacopée Française pour les médicaments. Les conditions de culture des microorganismes sont classiques et adaptés aux différentes souches utilisées qui sont celles recommandées pour l'étude des agents antiseptiques d'une manière générale et reflètent celles que l'on retrouve classiquement au niveau de l'oeil.

On détermine la concentration en préparation solide (à 3% d'alcool dichloro-2,4 benzylique) nécessaire pour détruire $10^5$ germes à 26°C ainsi que le temps nécessaire pour cette destruction. Les résultats sont donnés dans le tableau suivant.

| Souches testées Référence | Concentration en préparation solide (mg/ml) | Temps (mn) pour détruire $10^5$ germes |
|---|---|---|
| Klebsiella pneumoniae | | |
| IP - 52 144 | 5 à 10 | < 5 |
| Branhamella catharalis | 5 | < 2 |
| Escherichia coli | | |
| IP 54 127 | 5 | < 2 |
| IP 54 117 | 10 | < 2 |
| K12 J53 | 5 à 10 | < 2 |
| RPL III TEMI | 5 | < 2 |
| RL 114 TEMII | 5 | < 2 |

| Souches testées<br>Référence | Concentration en<br>préparation solide<br>(mg/ml) | Temps (mn) pour<br>détruire $10^5$ germes |
|---|---|---|
| **Staphylococcus aureus** | | |
| IP 52 148 | 5 | < 2 |
| IP 53 154 | 5 | < 2 |
| IP 52 150 | 5 | < 2 |
| **Staphylococcus epidermidis** | | |
| IP 6821 | 5 à 10 | < 2 |
| **Pseudomonas aeruginosa** | | |
| A 22 | 5 à 10 | < 2 |
| **Candida tropicalis** | | |
| IP 4 352 | 5 | < 10 |
| IP 20123 | 5 | < 10 |
| 1439 - 83 | 5 | < 10 |
| **Candida albicans** | | |
| 1180 - 79 | 10 | < 10 |
| **Streptococcus faecalis** | | |
| D 5855 | 15 | < 2 |
| D 5348 | 15 | < 2 |
| **Streptococcus pyogenes** | | |
| A 54 - 5 | 10 | < 2 |
| **Streptococcus equisimilis** | | |
| C 56 - 89 | 10 | < 2 |
| **Streptococcus sp.** | | |
| Groupe G 55120 | 10 | < 2 |

**Revendications**

1. Procédé de décontamination de lentilles de contact, caractérisé en ce qu'on immerge les lentilles de contact dans un solvant aqueux dans lequel est dissous, en tant qu'ingrédient actif de décontamination, de l'alcool dichloro-2,4 benzylique.

2. Procédé selon la revendication 1, caractérisé en ce que le solvant aqueux utilisé est une solution isotonique de chlorure de sodium dans l'eau.

3. Procédé selon la revendication 1 ou 2, caractérisé en ce que l'alcool dichloro-2,4 benzylique est dissous dans le solvant aqueux au moment de l'emploi.

EP 0 409 703 B1

4. Préparation solide pour la décontamination de lentilles de contact, caractérisée en ce qu'elle contient, en tant qu'ingrédient actif de décontamination, de l'alcool dichloro-2,4 benzylique.

5. Préparation selon la revendication 4, caractérisée en ce qu'elle contient de 2 à 5% en poids d'alcool dichloro-2,4 benzylique.

6. Préparation selon la revendication 4 ou 5, caractérisée en ce qu'elle contient, en tant qu'agent de lubrification facilitant la mise en forme galénique de la préparation solide, de l'acide benzoïque, ou du benzoate de sodium, ou un autre sel de métal alcalin ou alcalino-terreux de l'acide benzoïque.

7. Préparation selon la revendication 6, caractérisée en ce qu'elle contient l'agent de lubrification dans un rapport en poids de 1/1 à 6/1 par rapport à l'alcool dichloro-2,4 benzylique.

8. Préparation selon l'une quelconque des revendications 4 à 7, caractérisée en ce qu'elle contient un agent détergent et un agent séquestrant.

9. Préparation selon l'une quelconque des revendications 4 à 8, caractérisée en ce qu'elle contient un agent mouillant.

10. Préparation selon l'une quelconque des revendications 4 à 9, sous forme de comprimés, caractérisée en ce qu'elle contient un agent de délitement.

11. Préparation selon la revendication 10, caractérisée en ce qu'elle a la composition suivante (% en poids) :

```
Alcool dichloro-2,4 benzylique         3    %
Benzoate de sodium                    9,2   %
Acide éthylènediaminetétraacétique    7,5   %
Laurylsulfate de sodium               3,2   %
Acide citrique                         35   %
Hydrogénocarbonate de sodium           41   %
Polyvinylpyrrolidone                  1,1   %
```

12. Solution de décontamination de lentilles de contact obtenue par dissolution dans un solvant aqueux d'une préparation solide selon l'une quelconque des revendications 1 à 11.

13. Solution selon la revendication 12, caractérisée en ce que le solvant aqueux est une solution isotonique de chlorure de sodium dans l'eau.

14. Solution selon la revendication 12 ou 13, caractérisée en ce qu'elle contient de 0,2 à 0,5 mg d'alcool dichloro-2,4 benzylique par ml.

**Patentansprüche**

1. Verfahren zur Entkeimung von Kontaktlinsen, **dadurch gekennzeichnet**, daß man die Kontaktlinsen in ein wässeriges Lösungsmittel eintaucht, in welchem als aktiver Entkeimungsbestandteil 2,4-Dichlorbenzylalkohol gelöst ist.

2. Verfahren gemäß Anspruch 1, **dadurch gekennzeichnet**, daß das verwendete wässerige Lösungsmittel eine isotonische Lösung von Natriumchlorid in Wasser ist.

3. Verfahren gemäß Anspruch 1 oder 2, **dadurch gekennzeichnet**, daß der 2,4-Dichlorbenzylalkohol in dem wässerigen Lösungsmittel im Zeitpunkt der Anwendung gelöst ist.

4. Festpräparat für die Entkeimung von Kontaktlinsen, **dadurch gekennzeichnet**, daß es als aktiven Entkeimungsbestandteil 2,4-Dichlorbenzylalkohol enthält.

6

5. Präparat gemäß Anspruch 4, **dadurch gekennzeichnet**, daß es von 2 bis 5 Gewichtsprozent 2,4-Dichlorbenzylalkohol enthält.

6. Präparat gemäß Anspruch 4 oder 5, **dadurch gekennzeichnet**, daß es als die Gleitfähigkeit erleichterndes Mittel das in galenische Form überführte Festpräparat aus Benzoesäure oder Natriumbenzoat oder einem anderen Alkalimetall- oder Erdalkalimetall-Salz der Benzoesäure enthält.

7. Präparat gemäß 6, **dadurch gekennzeichnet**, daß es das Gleitmittel in einem Gewichtsverhältnis von 1/1 bis 6/1 zu 2,4-Dichlorbenzylalkohol enthält.

8. Präparat gemäß einem der Ansprüche 4 bis 7, **dadurch gekennzeichnet**, daß es ein Reinigungsmittel und ein Maskierungsmittel enthält.

9. Präparat gemäß einem der Ansprüche 4 bis 8, **dadurch gekennzeichnet**, daß es ein Netzmittel enthält.

10. Präparat gemäß einem der Ansprüche 4 bis 9 in Form von Pastillen, **dadurch gekennzeichnet**, daß es ein Zerfallmittel enthält.

11. Präparat gemäß Anspruch 10, **gekennzeichnet** durch die folgende Zusammensetzung (in Gewichtspro-zenten):

```
2,4-Dichlorbenzylalkohol ....................   3    %
Natriumbenzoat ...............................  9,2  %
Ethylendiamintetraessigsäure ................  7,5  %
Natriumlaurylsulfat ..........................  3,2  %
Citronensäure ................................ 35    %
Natriumhydrogencarbonat ...................... 41    %
Polyvinylpyrrolidon ..........................  1,1  %
```

12. Entkeimungslösung von Kontaktlinsen, erhalten durch Auflösung in einem wässerigen Lösungsmittel eines Festpräparats gemäß einem der Ansprüche 1 bis 11.

13. Lösung gemäß Anspruch 12, **dadurch gekennzeichnet**, daß das wässerige Lösungsmittel eine isotonische Lösung von Natriumchlorid in Wasser ist.

14. Lösung gemäß Anspruch 12 oder 13, **dadurch gekennzeichnet**, daß sie von 0,2 bis 0,5 mg 2,4-Dichlorbenzylalkohol pro ml enthält.

**Claims**

1. Method of decontaminating contact lenses, characterised in that the contact lenses are immersed in an aqueous solvent in which 2,4-dichlorobenzyl alcohol is dissolved as the active decontamination ingredient.

2. Method according to Claim 1, characterised in that the aqueous solvent used is an isotonic solution of sodium chloride in water.

3. Method according to Claim 1 or 2, characterised in that the 2,4-dichlorobenzyl alcohol is dissolved in the aqueous solvent at the time of use.

4. Solid preparation for decontaminating contact tenses, characterised in that it contains, as the active decontamination ingredient, 2,4-dichlorobenzyl alcohol.

5. Preparation according to Claim 4, characterised in that it contains 2 to 5% by weight of 2,4-dichlorobenzyl alcohol.

6.  Preparation according to Claim 4 or 5, characterised in that it contains benzoic acid, or sodium benzoate, or another alkaline metal or alkaline-earth salt of benzoic acid, as a lubrication agent facilitating the galenic formation of the solid preparation.

7.  Preparation according to Claim 6, characterised in that it contains the lubrication agent in a ratio by weight of 1:1 to 6:1 with respect to the 2,4-dichlorobenzyl alcohol.

8.  Preparation according to any one of Claims 4 to 7, characterised in that it contains a detergent and a sequestering agent.

9.  Preparation according to any one of Claims 4 to 8, characterised in that it contains a wetting agent.

10. Preparation according to any one of Claims 4 to 9, in the form of tablets, characterised in that it contains an exfoliation agent.

11. Preparation according to Claim 10, characterised in that it has the following composition (% by weight)

| | |
|---|---|
| 2,4-dichlorobenzyl alcohol | 3 % |
| Sodium benzoate | 9.2% |
| Ethylene diamine tetracetic acid | 7.5% |
| Sodium lauryl sulphate | 3.2% |
| Citric acid | 35 % |
| Sodium hydrogen carbonate | 41 % |
| Polyvinyl pyrrolidone | 1.1% |

12. Solution for decontaminating contact lenses obtained by dissolving, in an aqueous solvent, a solid preparation according to any one of Claims 1 to 11.

13. Solution according to Claim 12, characterised in that the aqueous solvent is an isotonic solution of sodium chloride in water.

14. Solution according to Claim 12 or 13, characterised in that it contains 0.2 to 0.5 mg of 2,4-dichlorobenzyl alcohol per ml.